(19) Europäisches Patentamt
European Patent Office
Office européen des brevets

(11) Veröffentlichungsnummer: **0 061 639**
**B1**

(12) **EUROPÄISCHE PATENTSCHRIFT**

(45) Veröffentlichungstag der Patentschrift: **29.05.85**

(51) Int. Cl.⁴: **C 07 D 279/16,** C 08 K 5/46

(21) Anmeldenummer: **82102070.8**

(22) Anmeldetag: **15.03.82**

(54) **Neue Stabilisatoren.**

(30) Priorität: **24.03.81 DE 3111513**

(43) Veröffentlichungstag der Anmeldung:
**06.10.82 Patentblatt 82/40**

(45) Bekanntmachung des Hinweises auf die Patenterteilung:
**29.05.85 Patentblatt 85/22**

(84) Benannte Vertragsstaaten:
**CH DE FR GB IT LI**

(56) Entgegenhaltungen:
**CH - A - 576 969**
**DE - A - 1 620 308**

(73) Patentinhaber: **BAYER AG, Konzernverwaltung RP Patentabteilung, D-5090 Leverkusen 1 Bayerwerk (DE)**

(72) Erfinder: **Uhrhan, Paul, Dr., Brunnenweg 27, D-5068 Odenthal (DE)**
Erfinder: **Krauthausen, Edmund, Dr., Franz-Grillparzer-Ring 4, D-5000 Köln 71 (DE)**
Erfinder: **Stahlke, Kurt-Rainer, Dr., Amselweg 7, D-5067 Kürten 2 (DE)**
Erfinder: **Quaas, Gerwolf, Bilharzstrasse 11, D-5000 Köln 80 (DE)**
Erfinder: **Ruetz, Lothar, Dr., Wilhelm-Busch-Strasse 37, D-4047 Dormagen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 061 639 B1

BUNDESDRUCKEREI BERLIN

**Beschreibung**

Die Erfindung betrifft neue Dihydrobenzothiazinverbindungen, deren Herstellung und deren Verwendung als Stabilisatoren und Co-Stabilisatoren gegen oxidativen und/oder thermischen Abbau von organischen Polymeren.

Bekanntlich unterliegen viele organische Polymere, wie z. B. Polyolefine, ABS-Polymerisate und verschiedene kautschukartige Polymere bei ihrer Lagerung und Verarbeitung, ebenso wie die daraus gefertigten Erzeugnisse bei ihrem Gebrauch einem Abbau unter dem Einfluß von Wärme, Sauerstoff, Ozon und/oder Licht. Hierbei kommt es zu einer Beeinträchtigung der physikalischen Eigenschaften, wie beispielsweise zur Verfärbung, Trübung, zu einer Rißbildung auf der Oberfläche, zu einer Verschlechterung der mechanischen Eigenschaften, z. B. der Zugfestigkeit. Um dies zu vermeiden, ist es üblich, Stabilisatoren zuzusetzen.

Die am besten wirkenden Antioxidantien sind aminische Verbindungen, z. B. p-Phenylendiaminderivate oder oligomeres 2,2,4-Trimethyl-1,2-dihydrochinolin. Jedoch verfärben sie die hiermit stabilisierten Produkte; sie führen zu einer mehr oder weniger ausgeprägten Kontaktverfärbung (J. Voigt »Die Stabilisierung der Kunststoffe gegen Licht und Wärme«, Springer-Verlag, Berlin—Heidelberg—New York 1966, insbesondere Seite 279—316). Daher können Aminstabilisatoren in der Praxis nur begrenzt eingesetzt werden, z. B. in Autoreifen, während man meist auf die weniger wirksamen phenolischen Stabilisatoren angewiesen ist.

So sind z. B. aus der FR-PS 1 344 437 spezielle Dihydrobenzothiazine als Antioxidantien bekannt. Jedoch ist deren Wirkung bei höheren Temperaturen nicht immer ganz befriedigend.

Es besteht also ein Bedarf an nichtverfärbenden Antioxidantien mit einer Wirkung vergleichbar der aminischer Stabilisatoren.

Generell beeinflussen phenolische und aminische Oxidationsinhibitoren vernetzbare Polymere ungünstig, weil die radikalische Vernetzung durch Übertragung von Wasserstoffatomen gestört wird. Dabei wird ein Teil der Oxidationsinhibitoren inaktiviert. Hinzu kommt noch, daß vernetzte Polymere oxidationsempfindlicher sind als unvernetzte, da sie noch langlebige Radikale (die als Startradikale für die thermooxidative Alterung fungieren) und eine erhöhte Konzentration an leicht oxidablen Gruppen enthalten. Die Oxidationsinhibitoren müssen also, obwohl sie durch die Vernetzungsreaktion in ihrer Wirksamkeit gemindert werden, höheren Anforderungen gerecht werden. Eine Erhöhung der Oxidationsinhibitorkonzentration führt jedoch auch zu einer stärkeren Beeinträchtigung der Vernetzung, so daß es schwierig ist, ausreichend stabilisierte Polymere mit hohem Vernetzungsgrad herzustellen.

Die erfindungsgemäßen Verbindungen der Formel I sind nichtverfärbende Stabilisatoren, mit einer Wirkung vergleichbar der aminischer Antioxidantien; sie beeinträchtigen die Vernetzung vernetzbarer Polymerer, insbesondere von Polyolefinen nicht.

Die erfindungsgemäßen Verbindungen entsprechen der allgemeinen Formel I

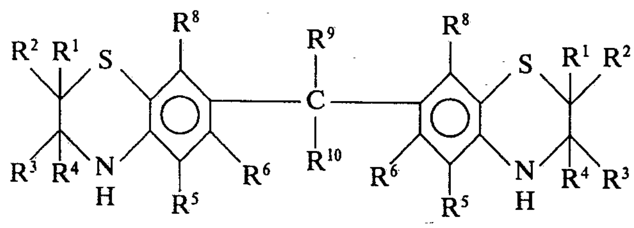

(I)

worin

| | |
|---|---|
| $R^1$ bis $R^4$ | gleich oder verschieden sind und |
| $R^1$ und $R^2$ | Wasserstoff oder niederes Alkyl mit 1 bis 4 C-Atomen und |
| $R^3$ und $R^4$ | niederes Alkyl mit 1 bis 4 C-Atomen bedeuten, |
| $R^5$ bis $R^8$ | Wasserstoff und |
| $R^9$ und $R^{10}$ | unabhängig voneinander für Wasserstoff, p-Chlorphenyl, lineares oder verzweigtes Alkyl mit 1 bis 12 C-Atomen, Cycloalkyl oder Cycloalkenyl mit 5 bis 8 C-Atomen stehen oder |
| $R^9$ und $R^{10}$ | zusammen mit dem C-Atom an das sie gebunden sind, einen Cycloalkylrest mit 5 bis 7 C-Atomen bilden. |

Bevorzugt stehen, unabhängig voneinander, $R^1$ und $R^2$ für Wasserstoff oder Methyl, $R^3$ und $R^4$ für Methyl und $R^5$ bis $R^8$ für Wasserstoff und $R^9$ und $R^{10}$ die oben angegebene Bedeutung haben.

Beispiele erfindungsgemäßer Verbindungen sind:

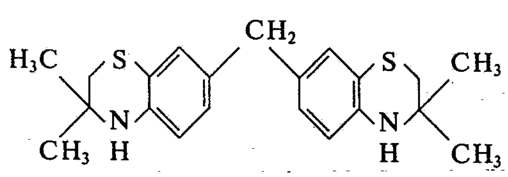

Di-(3,3-dimethyl-2,3-dihydro-4H-1,4-benzothiazin-7-yl)methan;
Di-(2,2,3,3-tetramethyl-2,3-dihydro-4H-1,4-benzothiazin-7-yl)methan
1,1-Di-(3,3-dimethyl-2,3-dihydro-4H-1,4-benzothiazin-7-yl)cyclohexan

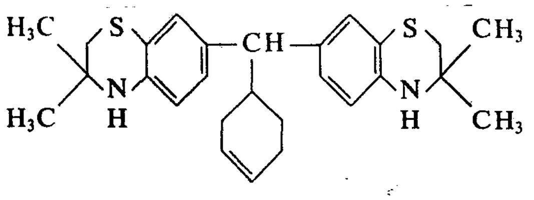

1-Cyclohex-3-enyl-1,1-di-(3,3-dimethyl-2,3-dihydro-4H-1,4-benzothiazin-7-yl)-methan
1-(4-Chlorphenyl)-1,1-di-(3,3-dimethyl-2-propyl-2,3-dihydro-4H-1,4-benzothiazin-7-yl)-methan

Die erfindungsgemäßen Verbindungen der Formel I lassen sich dadurch herstellen, daß man eine Verbindung der Formel II

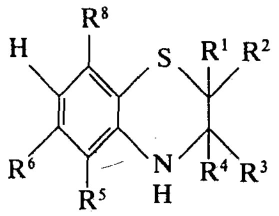

(II)

worin die allgemeinen Reste die oben angegebene Bedeutung haben,
mit einer Carbonylverbindung der Formel III

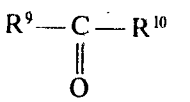

(III)

worin die allgemeinen Reste die oben angegebene Bedeutung haben,
im Molverhältnis von 10 : 1 bis 1 : 1, gegebenenfalls in einem inerten organischen Lösungsmittel und gegebenenfalls in Anwesenheit eines Katalysators bei Temperaturen von ca. 0°C bis ca. 200°C umsetzt.

Vorteilhaft ist der Einsatz eines sauren Katalysators, bevorzugt die Verwendung einer Mineralsäure. Die Reaktion kann in einem Lösungsmittel wie Ether, Alkohol, Toluol oder Tetrachlorkohlenstoff durchgeführt werden. Es ist auch möglich, die Reaktion ohne Lösungsmittel durchzuführen. Das Mol-Verhältnis zwischen der Verbindung der Formel II und der Carbonylverbindung III kann von 10 : 1 bis 1 : 1 variieren; vorzugsweise beträgt das Mol-Verhältnis 4 : 1 bis 2 : 1. Um die Reaktionszeit zu verkürzen, werden erhöhte Temperaturen von 50°C bis 150°C bzw. Siedetemperatur bevorzugt. Aus dem gleichen Grunde ist es in manchen Fällen vorteilhaft, die Komponenten bei erhöhtem Druck umzusetzen.

Die erfindungsgemäßen Verbindungen eignen sich zur Stabilisierung von organischen Polymeren gegen den oxidativen Abbau in der Wärme, insbesondere von Kunststoffen, wie Polyolefinen, ABS-Polymerisaten, schlagfestem Polystyrol, Polyestern, Polyamiden, Polycarbonaten, Polyurethanen, Naturkautschuk, Synthesekautschuken, wie Butadien-Styrol-Copolymere, Polybutadien, Polyisopren, Ethylen-Propylen-Dien-Terpolymere (EPDM-Kautschuk), Butylkautschuk, chloriertem und chlorsulfoniertem Polyethylen, Nitrilkautschuk.

Ein bevorzugtes Einsatzgebiet für die erfindungsgemäßen Verbindungen sind radikalisch vernetzte Polymere, insbesondere vernetztes Polyethylen, aber auch Ethylen/Propylen-Copolymerisate und EPDM-Kautschuk, chloriertes und chlorsulfoniertes Polyethylen.

Die erfindungsgemäßen Verbindungen werden gewöhnlich in Mengen von 0,001 bis 20%, bevorzugt in Mengen von 0,05 bis 5% — bezogen auf das Gesamtgewicht des zu stabilisierenden Polymeren — eingesetzt. Die Verbindungen können direkt oder über einen Masterbatch, der z. B. 30 bis 90% einer Verbindung der Formel I enthält, dosiert werden. Die Methode der Verwendung von Masterbatches ist allgemein bekannt. Die stabilisierten organischen Materialien können nur Verbindungen der Formel I enthalten oder noch zusätzlich andere Hilfsstoffe zur Verbesserung der Eigenschaften. Solche Hilfsmittel sind beispielsweise weitere Stabilisatoren oder Co-Stabilisatoren gegen die Zerstörung durch Hitze und Sauerstoff oder UV-Licht. Beispiele solcher Co-Stabilisatoren sind Dilauryl- und Distearylthiodipropionat, 2-Merkaptobenzimidazol, 2,6-Di-tert.-butyl-p-kresol, o-Hydroxybenzophenon und dergl. Andere Additive wie Korrosions- und Rostschutzmittel, Dispergiermittel, Emulgatoren, Weichmacher, Gleitmittel, Flamm- und Ozonschutzmittel, Metalldesaktivatoren, Treibmittel, Haftmittel, Farbstoffe, Pigmente, Füllstoffe, Ruß, Beschleuniger und Vernetzungsmittel können ebenfalls zugegen sein.

Die Erfindung betrifft auch die organischen Materialien, welche eine erfindungsgemäße Verbindung der Formel I enthalten, sowie auch ein Mittel, welches eine Verbindung der Formel I enthält.

Anhand folgender Beispiele soll die Erfindung noch näher erläutert werden.

Beispiele

Beispiel 1

2,869 kg (16 Mol) 3,3-Dimethyl-2,3-dihydro-4H-1,4-benzothiazin, 5 kg Methanol und 46 g (0,45 Mol) konzentrierte Schwefelsäure werden vorgelegt und bei 30° C 327 g (4 Mol) 37%ige wäßrige Formaldehyd-Lösung tropfenweise zugefügt. Man kocht 1,5 Std. unter Rückfluß, versetzt mit 40 g (1 Mol) Natriumhydroxid, und kocht weitere 3 Std. unter Rückfluß. Man saugt nach dem Abkühlen ab, wäscht den Nutschrückstand mit Methanol und Wasser und trocknet im Umluftschrank.

Man erhält 1,26 kg Di-(3,3-dimethyl-2,3-dihydro-4H-1,4-benzothiazin-7-yl)-methan mit Schmelzpunkten 179—81° C. Das Massenspektrum zeigt ein Molekülion bei 370 (100% rel. Intensivität) und als Hauptfragmente 355 (90%), 192 (23%), 170 (20%) (Stabilisator A).

Beispiel 2

Tropft man analog Beispiel 1 Isobutyraldehyd anstelle von Formaldehyd zu, so erhält man 2-Methyl-1-(3,3-dimethyl-2,3-dihydro-4H-1,4-benzothiazin-7-yl)-1-[3,3-dimethyl-5-(2-methyl-1-propenyl)-2,3-dihydro-4H-1,4-benzothiazin-7-yl]-propan als zähes Harz.

Das Massenspektrum zeigt ein Molekülion bei 466 (24% rel. Intensität) und als Hauptfragmente 423 (74%), 412 (14%), 369 (100%), 272 (19%), 178 (7%) (Stabilisator B).

Beispiel 3

Entsprechend Beispiel 1 erhält man aus 12 g 2,2,3,3-Tetramethyl-2,3-dihydro-4H-1,4-benzothiazin und 2,4 g 37%ige Formaldehyd-Lösung 8,2 g Di-(2,2,3,3-tetramethyl-2,3-dihydro-4H-1,4-benzothiazin-7-yl)-methan, dessen Massenspektrum ein Molekülion bei 426 (94% rel. Intensität) und die Hauptfragmente 411 (18%) und 383 (100%) zeigt.

Daneben enthält das Produkt noch geringe Mengen 3 und 4 (2,2,3,3-Tetramethyl-2,4-dihydro-4H-1,4-benzothiazin)-Kerne enthaltende Verbindungen mit den Molmassen 645 und 864.

Beispiel 4

90 g (0,5 Mol) 3,3-Dimethyl-2,3-dihydro-4H-1,4-benzothiazin, 500 ml Toluol, 3,2 g konzentrierte Schwefelsäure und 27,5 g (0,25 Mol) Tetrahydrobenzaldehyd werden 5 Std. unter Rückfluß gekocht, mit Wasser gewaschen, die organische Phase getrocknet, und das Lösungsmittel im Vakuum entfernt.

Unumgesetztes 3,3-Dimethyl-2,3-dihydro-4H-1,4-benzothiazin wird im Hochvakuum abdestilliert, und man erhält als Rückstand 1-Cyclohex-3-enyl-1,1-di-(3,3-dimethyl-2,3-dihydro-4H-1,4-benzothiazin-7-yl)-methan als dunkelbraunes sprödes Harz.

Das Massenspektrum zeigt als Molekülion 450 (26% rel. Intensität) und als Hauptfragmente 370 (27%), 369 (100%) (Stabilisator C).

Beispiel 5

Beispiel 5 zeigt, daß die erfindungsgemäßen Verbindungen in Naturkautschuk phenolischen Stabilisatoren überlegen und in ihrer Stabilisatorwirkung mit dem aminischen Alterungsschutzmittel 2,2,4-Trimethyl-1,2-dihydrochinolin-Oligomer vergleichbar sind.

Folgende Mischung wurde auf der Walze hergestellt:

| | Gew.-Teile |
|---|---|
| Helle Crepe | 100,0 |
| Zinkoxid | 10,0 |
| Stearinsäure | 1,0 |
| Titandioxid | 10,0 |
| Blanc fixe | 60,0 |
| Tetramethylthiuramdisulfid | 0,5 |
| Schwefel | 2,0 |
| Stabilisator, wie in Tabelle 1 angegeben | |

Es wurde 15 Min. bei 130° C in der Presse vulkanisiert. Das erhaltene Vulkanisat wurde einer Alterung in der Sauerstoffbombe (nach Bierer-Davis) bei 21 bar Sauerstoff 70° C ausgesetzt (DIN 53 508).

Die Ergebnisse sind in Tabelle 1 zusammengefaßt.

Tabelle 1

| | ohne Stabilisator (Vergl. 1) | Gemisch alkyl. u. aralkyl. Phenole (Vergl. 2) | 2,2,4-Trimethyl-1,2-dihydrochinolin-Oligomer (Vergl. 3) | A | B | C |
|---|---|---|---|---|---|---|
| Gew.-Teile | – | 1 | 1 | 1 | 1 | 1 |
| Vor der Alterung | | | | | | |
| Festigkeit (MPa) | 22,7 | 23,1 | 23,8 | 20,4 | 21,5 | 23,3 |
| Dehnung (%) | 730 | 730 | 710 | 730 | 780 | 720 |
| Modul bei 450% Dehnung (MPa) | 8,1 | 8,0 | 9,6 | 5,9 | 6,9 | 9,0 |
| Elastizität (%) | | | | | | |
| bei 20° C | 74 | 74 | 75 | 69 | 69 | 72 |
| bei 70° C | 79 | 81 | 81 | 72 | 75 | 77 |
| Härte (Shore A) | | | | | | |
| bei 20° C | 52 | 52 | 54 | 47 | 47 | 52 |
| bei 70° C | 52 | 52 | 53 | 47 | 44 | 52 |
| Nach 7 Tagen Alterung | | | | | | |
| Festigkeit (MPa) | 5,0 | 14,6 | 16,8 | – | 16,8 | 16,3 |
| Dehnung (%) | 470 | 600 | 580 | – | 650 | 590 |
| Modul bei 450% Dehnung (MPa) | – | 8,9 | 11,5 | – | 8,9 | 10,5 |
| Elastizität (%) | | | | | | |
| bei 20° C | – | 65 | 72 | – | 63 | 63 |
| bei 70° C | 50 | 69 | 79 | – | 70 | 71 |
| Härte (Shore A) | | | | | | |
| bei 20° C | 47 | 52 | 56 | – | 48 | 54 |
| bei 70° C | 35 | 50 | 55 | – | 47 | 51 |
| Nach 14 Tagen Alterung | | | | | | |
| Festigkeit (MPa) | zerstört | 9,3 | 14,4 | 13,4 | 13,5 | 12,7 |
| Dehnung (%) | zerstört | 560 | 570 | 570 | 620 | 580 |

Tabelle 1 (Fortsetzung)

| | ohne Stabilisator (Vergl. 1) | Gemisch alkyl. u. aralkyl. Phenole (Vergl. 2) | 2,2,4-Trimethyl-1,2-dihydrochinolin-Oligomer (Vergl. 3) | A | B | C |
|---|---|---|---|---|---|---|
| Modul bei 450% Dehnung (MPa) | | 7,1 | 10,4 | 8,3 | 8,9 | 9,3 |
| Elastizität (%) | | | | | | |
| bei 20° C | | 47 | 66 | 60 | 58 | 57 |
| bei 70° C | | 56 | 75 | 70 | 66 | 70 |
| Härte (Shore A) | | | | | | |
| bei 20° C | | 46 | 54 | 48 | 47 | 52 |
| bei 70° C | | 38 | 53 | 46 | 45 | 50 |
| Nach 21 Tagen Alterung | | | | | | |
| Festigkeit (MPa) | zerstört | zerstört | 11,9 | 10,5 | 11,4 | 8,0 |
| Dehnung (%) | zerstört | zerstört | 540 | 550 | 600 | 520 |
| Modul bei 450% Dehnung (MPa) | | | 9,8 | 7,0 | 7,7 | 7,1 |
| Elastizität (%) | | | | | | |
| bei 20° C | | | 55 | 57 | 51 | 49 |
| bei 70° C | | | 63 | 66 | 58 | 58 |
| Härte (Shore A) | | | | | | |
| bei 20° C | | | 53 | 49 | 45 | 49 |
| bei 70° C | | | 50 | 47 | — | 40 |

Vulkanisate mit den in Tabelle 1 angegebenen Vergleichssubstanzen 1 und 2, mit 2,2'-Methylen-bis-6-tert.-butyl-4-methylphenol (Vergleich 4) und mit Stabilisator A zeigten vor Belichtung keinerlei Verfärbung, während Vulkanisate mit Vergleichssubstanz 3 schon rosa verfärbt waren. Nach 2 Monaten Tagesbelichtung war die Farbe der Vulkanisate mit den Vergleichssubstanzen 1 und 2 weiß geblieben, mit Stabilisator A schwach cremefarbig, mit Vergleichssubstanz 4 rosa und im Vergleich 3 dagegen hellbraun geworden. Die erfindungsgemäßen Verbindungen liegen also in der Verfärbung im Bereich der phenolischen Antioxidantien (Vergleich 1, 2 und 4) und verfärben die Vulkanisate deutlich weniger als aminische Alterungsschutzmittel (Vergleich 3).

Beispiel 6

Es wurde eine EPDM-Kabel-Mischung folgender Zusammensetzung hergestellt:

Tabelle 2

| | Gew.-Teile | Gew.-Teile |
|---|---|---|
| EPDM-Kautschuk[1]) | 50 | 50 |
| EPDM-Kautschuk[2]) | 50 | 50 |
| Kaolin | 150 | 150 |
| Vinylsilan | 1 | 1 |
| Polyethylen-Ozonschutzwachs | 10 | 10 |
| 2,2,4-Trimethyl-1,2-dihydrochinolin, polym. | 1 | — |
| Stabilisator A | — | 1 |
| Paraffin | 5 | 5 |
| Mineralölweichmacher | 30 | 30 |
| Triallylcyanurat | 1,5 | 1,5 |
| Zinkoxid | 5 | 5 |
| Dicumylperoxid 40%ig | 5 | 5 |
| Vor der Alterung: | | |
| Festigkeit (MPa) | 7 | 6,1 |
| Bruchdehnung (%) | 310 | 300 |
| Spannung bei 100% Dehnung (MPa) | 3 | 3,1 |
| Spannung bei 300% Dehnung (MPa) | 7 | 6,1 |
| Härte bei 20° C (Shore A) | 72 | 69 |
| Nach 10 Tagen Alterung bei 100° C Heißluft | | |
| Festigkeit (MPa) | 6,9 | 6,3 |
| Bruchdehnung (%) | 310 | 300 |
| Härte bei 20° C (Shore A) | 70 | 69 |
| Nach 7 Tagen Alterung bei 135° C in Heißluft | | |
| Festigkeit (MPa) | 8,8 | 7,5 |
| Bruchdehnung (%) | 330 | 290 |
| Härte bei 20° C (Shore A) | 77 | 74 |

Man ersieht aus Tabelle 2, daß der Stabilisator A ein ebenso gutes Alterungsschutzmittel ist wie das schwach verfärbende 2,2,4-Trimethyl-1,2-dihydrochinolin-Oligomer.

[1]) statistisches EPDM mit Ethylidennorbornen als Terkomponente und einer Mooney-Viskosität ML 1+4 bei 100° C von 70.
[2]) Sequenz-EPDM mit hoher Polymerrohfestigkeit, Ethylidennorbornen als Terkomponente und einer Mooney-Viskosität ML 1+4 bei 100° C von 85.

Beispiel 7

Es wurde folgende Naturlatexmischung hergestellt:

| | Gew.-Teile |
|---|---|
| Naturlatex | 167 |
| 3-Benzyl-4-hydroxy-biphenyl-polyglykolether 20%ig | 1 |
| Zinkoxid aktiv | 2,5 |
| Kolloider Schwefel | 1,8 |
| Zink-N-diethyldithiocarbamat | 0,8 |
| Methylen-bis(naphthalinsulfonsäure) 5%ig | 7,9 |
| Alterungsschutzmittel, wie in Tabelle 3 angegeben. | |

Die Herstellung der Dispersion erfolgte entweder mit Kugelmühle oder mit dem Ultraturax. Die Proben wurden 15 Min. bei 110° C vulkanisiert und einer Heißluftalterung bei 100° C unterworfen. Die Ergebnisse sind in Tabelle 3 zusammengefaßt.

Tabelle 3

| | Vergl. 1 | Vergl. 2 | Vergl. 3 | Vergl. 4 | Beisp. A | Beisp. B | Vergl. 5 | Vergl. 6 |
|---|---|---|---|---|---|---|---|---|
| 2,2,4-Trimethyl-1,2-di-hydrochinolin (Gew.-Tle) | 1 | 1 | 0,5 | 0,5 | – | – | – | – |
| 2-Merkaptobenzimidazol (Gew.-Tle) | – | – | 0,5 | 0,5 | 0,5 | 0,5 | – | – |
| Stabilisator A | – | – | – | – | 0,5 | 0,5 | – | – |
| Styrolisiertes Diphenylamin (Gew.-Tle) | – | – | – | – | – | – | 1 | – |
| Dispersion mit Kugelmühle | + | – | + | – | + | – | + | + |
| Dispersion mit Ultraturax | – | + | – | + | – | + | – | – |
| Lagerbeständigkeit der Mischung nach 14 Tagen | etwas stippig | etwas stippig | thixotrop | thixotrop u. etwas stippig | einwand-frei | sedimentiert n. d. Aufrühren, etw. stippig | einwand-frei | einwand-frei |
| Vor der Alterung | | | | | | | | |
| Zugfestigkeit (Kp/cm²) | 193 | 240 | 187 | 243 | 204 | 199 | 234 | 195 |
| Bruchdehnung (%) | 685 | 695 | 760 | 705 | 745 | 710 | 695 | 720 |
| Modul bei 500% Dehnung (Kp/cm²) | 52 | 50 | 27 | 40 | 39 | 41 | 44 | 29 |
| Nach 3 Tagen Alterung | | | | | | | | |
| Zugfestigkeit (Kp/cm²) | 47 | 25 | 100 | 77 | 55 | 78 | 34 | 8 |
| Bruchdehnung (%) | 430 | 465 | 555 | 430 | 565 | 485 | 555 | 260 |
| Modul bei 500% Dehnung (Kp/cm²) | – | – | 41 | – | – | – | – | – |
| Nach 6 Tagen Alterung | | | | | | | | |
| Zugfestigkeit (Kp/cm²) | 10 | 8 | 21 | 13 | 36 | 17 | 10 | 7 |
| Bruchdehnung (%) | 140 | 90 | 270 | 195 | 380 | 305 | 190 | 70 |
| Modul bei 500% Dehnung (Kp/cm²) | – | – | – | – | – | – | – | – |

Die Tabelle 3 dokumentiert die Überlegenheit der erfindungsgemäßen Verbindung (Stabilisator A) gegenüber den Vergleichssubstanzen bezüglich der Alterungsschutzwirkung und der Dispergierbarkeit im Latex.

## Beispiel 8

In einem für Standardverwendung normal stabilisiertem ABS-Handelsprodukt mit dem Monomerenverhältnis Acrylnitril zu Butadien zu Styrol = 20 : 32 : 48 Gew.-% und einem mittleren Molekulargewicht der SAN-Harzphase von 110 000 bis 160 000 wurden mittels eines 3-kg-Laborinnenkneters vom Banbury-Typ zusätzlich die in der Tabelle 5 angegebenen Stabilisatormengen eingearbeitet und daraus Normkleinstäbe gespritzt.

Es wurde der Erhalt der Schlagzähigkeit $a_n$ nach DIN 53 453 vor und nach einer Hitzeeinwirkung von 4 Min. unter dem Heizaggregat einer Vakuumwarmformanlage (Illig U 60; Abstand Normkleinstaboberfläche — IR-Strahler = 15 cm), sowie die Kerbschlagzähigkeit vor der Hitzebehandlung bestimmt. Die Ergebnisse der Tabelle 4 zeigen die gute Stabilisatorwirkung der erfindungsgemäßen Verbindung.

Tabelle 4

| Versuch | Stabilisatorzusatz | Schlagzähigkeit $a_n$ vor Hitzealterung | nach Hitzealterung | Kerbschlagzähigkeit $a_k$ (kJ/m²) |
|---|---|---|---|---|
| A | — | ungebrochen | 37 kJ/m² | 17 |
| B | 0,2% 2,2'Dihydroxy-3,3'-dicyclohexyl-5,5'-dimethyl-diphenylmethan (Stand der Technik) | ungebrochen | ungebrochen | 20 |
| C | 0,5% desgl. | ungebrochen | ungebrochen | 18 |
| D | 0,2% Stabilisator A | ungebrochen | ungebrochen | 19 |
| E | 0,5% desgl. | ungebrochen | ungebrochen | 19 |

## Beispiel 9

In Hochdruckpolyethylen-Basisharz für LDPE-Kabelisolierung (MI=2,6 g/10 min. bei 190°C; d=0,922 g/cm³) wurden nach Einarbeitung der angegebenen Stabilisatormengen und 1,5% Ditert.-butylperoxid 1 mm starke Preßplatten hergestellt, die bei 135°C vernetzt wurden. Anschließend stanzte man Normstäbe S2 und unterwarf sie einer Heißluftalterung bei 150°C.

Die Ergebnisse sind in Tabelle 5 wiedergegeben und zeigen den Vorteil der erfindungsgemäßen Verbindung.

Tabelle 5

| | Vor Alterung bei 150°C | Nach 10 Tg. | 20 Tg. | 40 Tg. | 100 Tg. |
|---|---|---|---|---|---|
| 0,3% 2,2,4-Trimethyl-1,2-dihydrochinolin-Oligomeres (Vergleich 1) Gelgehalt: 91% | | | | | |
| Reißfestigkeit (MPa) | 21,4 | 10,3 | 6,2 | zerstört | zerstört |
| Dehnung (%) | 445 | 218 | — | — | — |

Tabelle 5 (Fortsetzung)

| | Vor Alterung bei 150° C | Nach 10 Tg. | 20 Tg. | 40 Tg. | 100 Tg. |
|---|---|---|---|---|---|
| 0,5% 2,2,4-Trimethyl-1,2-dihydrochinolin-Oligomeres (Vergleich 2) Gelgehalt: 92% | | | | | |
| Reißfestigkeit (MPa) | 18,9 | 14,2 | 12,9 | 4,8 | zerstört |
| Dehnung (%) | 457 | 406 | 316 | 32 | — |
| 0,3% Stabilisator A Gelgehalt: 89% | | | | | |
| Reißfestigkeit (MPa) | 27,8 | 20,2 | 17,4 | 9,5 | 8,4 |
| Dehnung (%) | 580 | 494 | 462 | 95 | 92 |
| 0,5% Stabilisator A Gelgehalt: 85% | | | | | |
| Reißfestigkeit (MPa) | 26,5 | 21,2 | 18,4 | 12 | 9 |
| Dehnung (%) | 582 | 519 | 484 | 347 | 136 |

Im sogenannten Hot-Set-Test nach VDE 0472 wurden folgende Ergebnisse erhalten:

Probe mit 0,5% Stabilisator A:

150° C/0,2 MPa: 68% Dehnung
200° C/0,2 MPa: 70% Dehnung

Probe mit 0,5% 2,2,4-Trimethyl-1,2-dihydrochinolin-Oligomeres (Vergleich):

150° C/0,2 MPa: 39% Dehnung
200° C/0,2 MPa: 26% Dehnung.

Auch in diesem Test zeigt die erfindungsgemäße Verbindung eine wesentlich bessere Wirkung.

Beispiel 10

In Ethylen-Vinylacetat-Copolymer-Basisharz für LDPE-Kabelisolierungen (MI = 25 g/10 min. bei 190° C; d = 0,930 g/cm$^3$) mit einem Vinylacetat-Gehalt von 9% wurden 0,5% des angegebenen Stabilisators eingearbeitet und daraus 1 mm starke Preßplatten hergestellt. Anschließend wurde mit einem van-der-Graaf-Generator mit einer Strahlendosis von 20 Mrad vernetzt und Normstäbe S2 ausgestanzt, die einer Heißluftalterung bei 150° C unterworfen wurden.

Die in Tabelle 6 wiedergegebenen Ergebnisse zeigen die Vorteile der erfindungsgemäßen Verbindung.

Tabelle 6

| | Nach 0 Tage Alterung | 20 Tagen Alterung | 60 Tagen Alterung |
|---|---|---|---|
| 0,5% 2,2,4-Trimethyl-1,2-dihydrochinolin-Oligomeres (Vergleich) Gelgehalt: 75% | | | |
| Reißfestigkeit (MPa): | 28 | 2,9 | zerstört |
| Dehnung (%): | 499 | 6 | — |
| 0,5% Stabilisator A Gelgehalt: 78% | | | |
| Reißfestigkeit (MPa) | 26,9 | 8,5 | 7,1 |
| Dehnung (%): | 549 | 209 | 80 |

Auch im Hot-Set-Test nach VDE 0472 zeigt sich die erfindungsgemäße Verbindung überlegen:

LDPE mit 0,5% 2,2,4-Trimethyl-1,2-dihydrochinolin-Oligomer (Vergleich):

| | |
|---|---|
| 150° C/0,2 MPa | 53% Dehnung |
| 200° C/0,2 MPa | gerissen |

LDPE mit 0,5% Stabilisator A:

| | |
|---|---|
| 150° C/0,2 MPa | 105% Dehnung |
| 200° C/0,2 MPa | 65% Dehnung |

Beispiel 11

In ein Hochdruckpolyethylen-Basisharz für LDPE-Kabelisolierungen wurden 0,5% Stabilisator, 1,5% Vinyltrimethoxysilan; 0,15% Diacumylperoxid und 0,05% Dibutylzinndilaurat eingearbeitet und 1 mm starke Preßplatten hergestellt. Nach Vernetzung durch 3stündiges Einwirken von kochendem Wasser wurden Normstäbe S2 ausgestanzt und einer Heißluftalterung bei 150° C unterworfen.

Die in Tabelle 7 zusammengestellten Ergebnisse zeigen die gute Wirkung der erfindungsgemäßen Verbindung.

Tabelle 7

| | Nach 0 Tage Alterung | 10 Tagen Alterung | 30 Tagen Alterung |
|---|---|---|---|
| 0,5% 2,2,4-Trimethyl-1,2-dihydrochinolin-Oligomer (Vergleich) | | | |
| Reißfestigkeit (MPa): | 15 | 15,3 | 6,7 |
| Dehnung (%): | 310 | 353 | 12 |
| 0,5% Stabilisator A | | | |
| Reißfestigkeit (MPa): | 18,2 | 17 | 6 |
| Dehnung (%): | 510 | 430 | 35 |

**Patentansprüche**

1. Verbindungen der allgemeinen Formel I

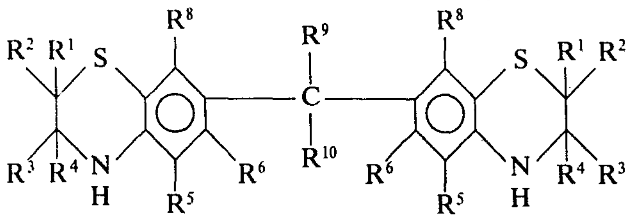

(I)

worin

$R^1$ bis $R^4$ gleich oder verschieden sind und
$R^1$ und $R^2$ Wasserstoff oder niederes Alkyl mit 1 bis 4 C-Atomen und
$R^3$ und $R^4$ niederes Alkyl mit 1 bis 4 C-Atomen bedeuten,
$R^5$ bis $R^8$ Wasserstoff und
$R^9$ und $R^{10}$ unabhängig voneinander für Wasserstoff, p-Chlorphenyl, lineares oder verzweigtes Alkyl mit 1 bis 12 C-Atomen, Cycloalkyl oder Cycloalkenyl mit 5 bis 8 C-Atomen stehen oder
$R^9$ und $R^{10}$ zusammen mit dem C-Atom an das sie gebunden sind, einen Cycloalkylrest mit 5 bis 7 C-Atomen bilden.

2. Verbindungen der Formel I, worin

$R^1$ und $R^2$ Wasserstoff oder Methyl sind,
$R^3$ und $R^4$ für Methyl stehen, und
$R^5$ bis $R^{10}$ die in Anspruch 1 angegebene Bedeutung haben.

3. Die Verbindung

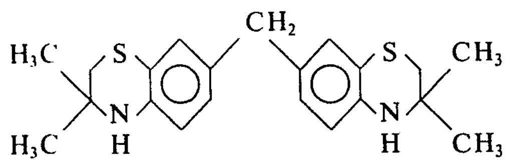

4. Verfahren zum Herstellen von Verbindungen der Formel I gemäß Anspruch 1, dadurch gekennzeichnet, daß man eine Verbindung der Formel II

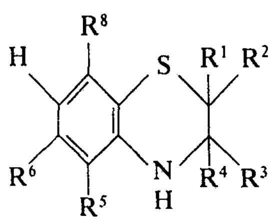

(II)

worin

$R^1$ bis $R^8$ die in Anspruch 1 angegebene Bedeutung haben,

gegebenenfalls mit Hilfe eines Katalysators und gegebenenfalls in einem inerten organischen Lösungsmittel mit einer Verbindung der Formel III

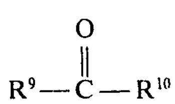

(III)

wobei

$R^9$ und $R^{10}$ die in Anspruch 1 angegebene Bedeutung haben,

im Molverhältnis von 10 : 1 bis 1 : 1 bei Temperaturen von 0—200° C umsetzt.

5. Verwendung der Verbindungen gemäß Anspruch 1 zum Stabilisieren von organischen Polymeren.

6. Verwendung gemäß Anspruch 5 zum Stabilisieren von Polyolefinen, ABS-Polymerisaten.

7. Verwendung gemäß Anspruch 5 zum Stabilisieren von radikalisch vernetzten Polymeren.

8. Verwendung nach Anspruch 5 zum Stabilisieren von vernetztem Polyethylen.

**Claims**

1. Compounds corresponding to the following general formula

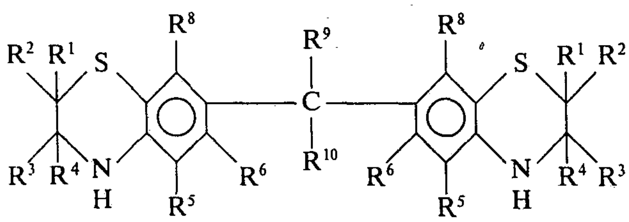

(I)

in which

| | |
|---|---|
| $R^1$ to $R^4$ | may be the same or different and |
| $R^1$ and $R^2$ | represent hydrogen or lower alkyl containing from 1 to 4 C-atoms and |
| $R^3$ and $R^4$ | represent lower alkyl containing from 1 to 4 C-atoms, |
| $R^5$ to $R^8$ | represent hydrogen and |
| $R^9$ and $R^{10}$ | independently of one another represent hydrogen, p-chlorophenyl, linear or branched alkyl containing from 1 to 12 C-atoms, cycloalkyl or cycloalkenyl containing from 5 to 8 C-atoms or |
| $R^9$ and $R^{10}$ | together with the C-atom to which they are attached, form a cycloalkyl radical containing from 5 to 7 C-atoms. |

2. Compounds corresponding to formula I in which

| | |
|---|---|
| $R^1$ and $R^2$ | represent hydrogen or methyl, |
| $R^3$ and $R^4$ | represent metyl and |
| $R^5$ and $R^{10}$ | are as defined in Claim 1. |

3. The compound

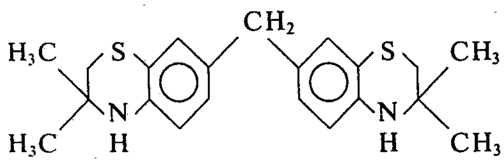

4. A process for producing the compounds corresponding to formula I claimed in Claim 1, characterized in that a compound corresponding to the following formula

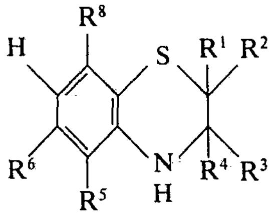

(II)

in which

$R^1$ to $R^8$ are as defined in Claim 1,

is reacted at 0—200° C with a compound corresponding to the following formula

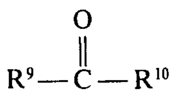

(III)

in which

$R^9$ and $R^{10}$ are as defined in Claim 1,

in a molar ratio of from 10 : 1 to 1 : 1, optionally in an inert organic solvent and optionally in the presence of a catalyst.

5. The use of the compounds claimed in Claim 1 for stabilizing organic polymers.
6. The use claimed in Claim 5 for stabilizing polyolefins, ABS-polymers.
7. The use claimed in Claim 5 for stabilizing radically crosslinked polymers.
8. The use claimed in Claim 5 for stabilizing crosslinked polyethylene.

**Revendications**

1. Composés de formule générale I

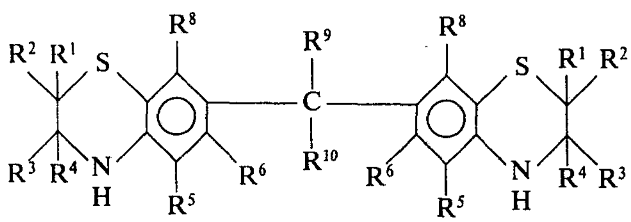

(I)

dans laquelle

| | |
|---|---|
| $R^1$ à $R^4$ | ont des significations identiques ou différentes, et |
| $R^1$ et $R^2$ | représentent l'hydrogène ou un groupe alkyle inférieur en C1—C4, et |
| $R^3$ et $R^4$ | représentent un groupe alkyle en C1—C4, |
| $R^5$ à $R^8$ | représentent l'hydrogène, et |
| $R^9$ et $R^{10}$ | représentent, indépendamment l'un de l'autre, l'hydrogène, un groupe p-chlorophényle, un groupe alkyle linéaire ou ramifié en C1—C12, cycloalkyle ou cycloalcényle en C5—C8, ou bien |
| $R^9$ et $R^{10}$ | forment ensemble et avec l'atome de carbone auquel ils sont reliés un groupe cycloalkyle en C5—C7. |

2. Composés de formule I, dans laquelle

| | |
|---|---|
| $R^1$ et $R^2$ | représentent l'hydrogène ou le groupe méthyle |
| $R^3$ et $R^4$ | représentent le groupe méthyle, et |
| $R^5$ à $R^{10}$ | ont les significations indiquées dans la revendication 1. |

3. Le composé

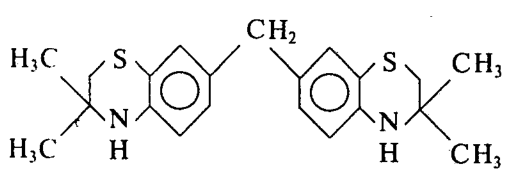

4. Procédé de préparation des composés de formule I selon la revendication 1, caractérisé en ce que

l'on fait réagir un composé de formule II

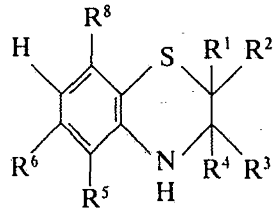

(II)

dans laquelle

$R^1$ à $R^8$ ont les significations indiquées dans la revendication 1,

éventuellement à l'aide d'un catalyseur et éventuellement dans un solvant organique inerte, avec un composé de formule III

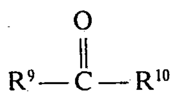

(III)

dans laquelle

$R^9$ et $R^{10}$ ont les significations indiquées dans la revendication 1,

à un rapport molaire de 10 : 1 à 1 : 1 et à des températures de 0 à 200° C.

5. Utilisation des composés selon la revendication 1, pour la stabilisation de polymères organiques.

6. Utilisation selon la revendication 5, pour la stabilisation de polyoléfines, de polymères ABS.

7. Utilisation selon la revendication 5, pour la stabilisation de polymères réticulés par des radicaux libres.

8. Utilisation selon la revendication 5, pour la stabilisation du polyéthylène réticulé.